# KRAUS · WEISERT & PARTNER

PATENTANWÄLTE
UND
ZUGELASSENE VERTRETER VOR DEM EUROPÄISCHEN PATENTAMT

Patentanwälte Thomas-Wimmer-Ring 15 D-8000 München 22

DR. WALTER KRAUS
DIPLOMCHEMIKER

DR.-ING. ANNEKÄTE WEISERT
DIPL.-ING.

JOHANNES SPIES
DIPL.-PHYS.

THOMAS-WIMMER-RING 15
D-8000 MÜNCHEN 22
TELEFON (0 89) 22 73 77
TELEX 5-212 156 kpat d
TELEFAX (0 89) 22 79 94

European Patent Office
Erhardtstraße 27

D-8000 Munich 2

EPA-EPO-OEB
MÜNCHEN
Empfang bestätigt
Receipt acknowledged
Accuse reception
CD

UNSERE NR.: 6244 AW/eg
OUR FILE:
Bitte in der Antwort stets angeben
Please refer to in your reply

July 26, 1988

Re: European patent application 87 118 854.6
Nihon Iyakuhin Kogyo Co., Ltd. et al.

Enclosed are in triplicate new pages 3, 4, 5 and 7 to 10 of the description which shall substitute the corresponding pages as now on file. Applicant discovered some portions to be amended in the description as indicated in handwriting in the originally filed pages, copies of which are enclosed to this letter.

It is politely submitted that according to Rule 88 EPC the amendments are allowable. According to Rule 88 EPC mistakes in any document filed with the European Patent Office may be corrected on request if the correction is obvious in the sense that it is immediately evident that nothing else would have been intended than what is offered as the correction.

European Patent Attorney

EPA EPO-OEB DG 1 Reçu: - 5-08-1988 05

Encls.
new pages 3-5 and
7-10
(in triplicate),
copies of pages amended
in handwriting

87118854.6



formula (II), i.e. dl-threo-2-hydroxy-3-(2-aminophenylthio)-3-(4-methoxyphenyl)-propionic acid. For example, Japanese Laid-Open Patent Publication No. 231065/1984 discloses a method in which optically active alpha-phenylethylamine is used as an optical resolving reagent. Japanese Laid-Open Patent ~~Publication No~~. 69756/1986 discloses a method in which 1-1,2-diphenylethylamine is used as an optical resolving reagent. However, the optical resolving reagents used in these methods are expensive and difficult to procure, and are unsuitable for use in industrial practice.

The present inventors have made investigations in order to develop a technique which can overcome this disadvantage. These investigations have unexpectedly led to the discovery that L-lysine or its acid salt such as L-lysine hydrochloride which is very cheap in price and easily available reacts easily with dl-threo-2-hydroxy-3-(2-aminophenylthio)-3-(4-methoxyphenyl)-propionic acid of formula (II) or its metal salt to form two optically active salts and effectively form ~~the wanted~~ difficultly-solvent-soluble salt of d-threo-2-hydroxy-3-(2-aminophenylthio)-3-(4-methoxy-phenyl)-propionic acid and L-lysine ~~while the salt~~ of the l-isomer remains dissolved in the solvent, and thus the novel salt of d-threo-2-hydroxy-3-(2-aminophenylthio)-3-(4-methoxyphenyl)-propionic acid of formula (1) and L-lysine can be industrially advantageously separated and recovered from the two salts.

It is an object of this invention therefore to provide L-lysine d-threo-2-hydroxy-3-(2-aminophenylthio)-3-(4-methoxyphenyl)-propionate which is useful, for example, as a synthesis intermediate for a known pharmaceutical compound, diltiazem hydrochloride, and which is not described in the prior literature.

Another object of this invention is to provide a process for producing the aforesaid salt.

Other objects of this invention along with its

advantages will become apparent from the following description.

The novel salt of d-threo-2-hydroxy-3-(2-aminophenylthio)-3-(4-methoxyphenyl)-propionic acid of formula (I) with L-lysine can be obtained by reacting dl-threo-2-hydroxy-3-(2-aminophenylthio)-3-(4-methoxyphenyl)-propionic acid and L-lysine or a metal salt of said propionic acid with an acid salt of L-lysine to form corresponding salts, and separating the difficultly-soluble salt from the resulting two optically active salts by utilizing the difference in solubility in a solvent between the two optically active salts.

The reaction of the dl-isomer of formula (II) or its metal salt with L-lysine or its acid salt can be carried out by contacting them with each other in a suitable resolving solvent. The resolving solvent may be an aqueous organic solvent, for example mixtures of water with lower alcohols such as methanol, ethanol and isopropanol, and a mixture of acetone and water. The amount of the resolving solvent may be properly varied, and is, for example, about 2 to about 5 times the volume of the dl-isomer of formula (II). The amount of L-lysine used is nearly equivalent, for example about 1 to about 1.2 equivalents, to the dl-isomer of formula (II).

Desirably, the reaction between the dl-isomer of formula (II) or its metal salt with L-lysine or its acid salt is carried out under heat. The two optically active salts formed by the reaction may be allowed to cool, or positively cooled, to crystallize the difficultly-soluble salt by utilizing the difference in solubility in a solvent between the two diastereomers. The difficultly-soluble salt is separated and obtained as L-lysine d-threo-2-hydroxy-3-(2-aminophenylthio)-3-(4-methoxyphenyl)-propionate.

The heating temperature may be a suitable temperature at which the dl-isomer of formula (II) or its

metal salt and L-lysine or its acid salt can dissolve in the resolving solvent. Usually, the reaction under refluxing conditions is preferred. Preferably, the reaction is carried out by using a metal salt of the dl-isomer of formula (II) such as its sodium salt and an acid salt of L-lysine such as L-lysine hydrochloride.

By reacting the dl-isomer of formula (II) with L-lysine or the metal salt of the dl-isomer of formula (II) and the acid salt of L-lysine in the manner described above, the corresponding salts are formed. The difficultly-soluble salt, L-lysine d-threo-2-hydroxy-3-(2-aminophenylthio)-3-(4-methoxyphenyl)-propionate, is separated by utilizing the difference in solubility in a solvent between the two resulting optically active salts. As desired, the resulting L-lysine salt may be recrystallized from a hydrous lower alcohol, for example, to increase its optical purity further. The L-lysine d-threo-2-hydroxy-3-(2-aminophenylthio)-3-(4-methoxyphenyl)-propionate may be converted to a free acid form of formula (I) by dissolving it in water, adding 10% hydrochloric acid for example, to adjust the pH of the system to about 2.5 to about 3.5, and collecting the precipitated crystals by filtration.

The starting dl-isomer of formula (II) is a known compound and can be produced, for example, by using the process described in Japanese Patent Publication No. 9383/1970.

One embodiment of producing the known pharmaceutical compound of formula (VI) from the L-lysine salt of the compound of formula (I) provided by this invention is schematically shown below.

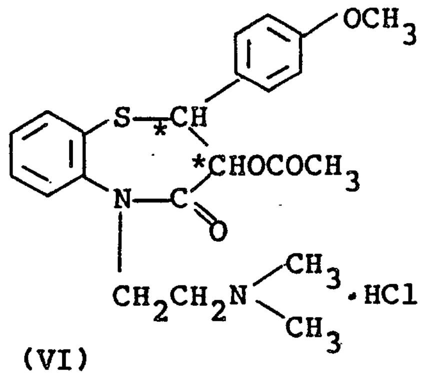

(VI)

In the above scheme, the compound of formula (III) may be obtained from the compound of formula (I) by, for example, using the process described in Japanese Patent Publication No. 8982/1971. The compound of formula (IV) may be obtained from the compound of formula (III) by, for example, using the process described in Japanese Patent Publication No. 43785/1971. The production of the pharmaceutical compound of formula (VI) from the compound of formula (IV) via the compound of formula (V) may be effected by, for example, using the process described in Japanese Laid-Open Patent Publication No. 32777/1985.

EXAMPLE 1

L-lysine d-threo-2-hydroxy-3-(2-aminophenylthio)-3-(4-methoxyphenyl)-propionate:-

To a solution of 27.5 g of sodium hydroxide in 140 ml of water were added 211 g of dl-threo-2-hydroxy-3-(2-aminophenylthio)-3-(4-methoxyphenyl)-propionate and 400 ml of methanol, and the mixture was heated to form a solution. Then, 125 g of L-lysine hydrochloride was added to the solution, and the mixture was heated under reflux. The solution was allowed to cool to room temperature. The precipitated crystals were collected by filtration, washed with methanol, recrystallized from 50% hydrous methanol, and dried to give 146.5 g of L-lysine d-threo-2-hydroxy-3-(2-aminophenylthio)-3-(4-methoxyphenyl)-propionate.

Yield: 95.2%

$[\alpha]_D^{20}$: +252.3° (c=0.5, water)

Melting point: 138-140°C

EXAMPLE 2

d-Threo-2-hydroxy-3-(2-aminophenylthio)-3-(4-methoxyphenyl)-propionic acid:-

146 g of the salt product obtained in Example 1 was dissolved in 500 ml of water and the solution was adjusted to pH 3.0 with 10% hydrochloric acid. The precipitated crystals were collected by filtration, washed with water, and dried to give 71.7 g of optically pure d-threo-2-hydroxy-3-(2-aminophenylthio)-3-(4-methoxyphenyl)-propionic acid.

Yield: 68.1%

$[\alpha]_D^{20}$: +350° (c=0.356, methanol)

Melting point: 137.5°C

EXAMPLE 3

L-lysine d-threo-2-hydroxy-3-(2-aminophenylthio)-3-(4-methoxyphenyl)-propionate:-

32 g of dl-threo-2-hydroxy-3-(2-aminophenylthio)-3-(4-methoxyphenyl)-propionic acid and 15.2 g of L-lysine were added to 35 ml of water and 100 ml of methanol. The mixture was heated to form a solution. The solution was allowed to cool to room temperature. The precipitated crystals were collected by filtration, washed with methanol, recrystallized from 50% hydrous methanol, and dried to give 20.5 g of L-lysine d-threo-2-hydroxy-3-(2-aminophenylthio)-3-(4-methoxyphenyl)-propionate.

Yield: 87.8%

$[\alpha]_D^{20}$: +252.0° (c=0.5, water)

Melting point: 138-140°C

What is claimed is:

1. A salt of d-threo-2-hydroxy-3-(2-aminophenylthio)-3-(4-methoxyphenyl)-propionic acid represented by the following formula (I)

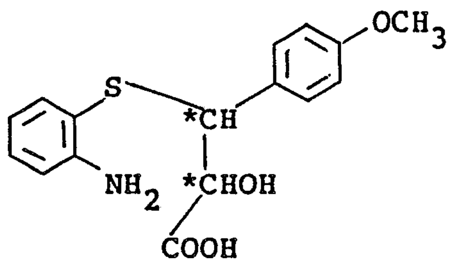

..... (I)

wherein C* represents the carbon atoms of the optically resolved molecules,

with L-lysine.

2. A process for producing a salt of d-threo-2-hydroxy-3-(2-aminophenylthio)-3-(4-methoxyphenyl)-propionic acid represented by the following formula (I)

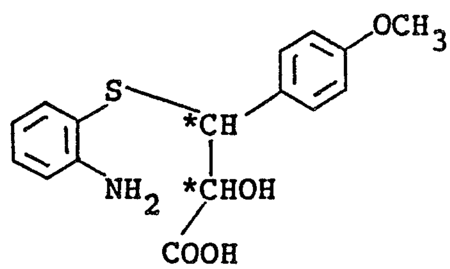

..... (I)

wherein C* represents the carbon atoms of the optically resolved molecules,

with L-lysine, which comprises reacting dl-threo-2-hydroxy-3-(2-aminophenylthio)-3-(4-methoxyphenyl)-propionic acid represented by the following formula (II)

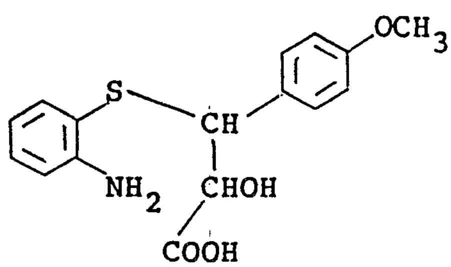

..... (II)

with L-lysine or a metal salt of said propionic acid with an acid salt of L-lysine to form corresponding salts, and separating the difficultly-soluble salt from the resulting two optically active salts by utilizing the difference in solubility in a solvent between the two optically active salts.

3. The process of claim 2 wherein the solvent is an aqueous organic solvent.

4. The process of claim 3 wherein the aqueous organic solvent is a mixture of a lower alcohol and water, or a mixture of acetone and water.

5. The process of claim 2 wherein about 1 to about 1.2 equivalents of L-lysine or its acid salt is used per equivalent of dl-~~threo-2-~~hydroxy-3-(2-aminophenylthio)-3-(4-methoxyphenyl)-propionic acid of formula (II) or its metal salt.

6. The process of claim 2 wherein the reaction of dl-threo-2-hydroxy-3-(2-aminophenylthio)-3-(4-methoxyphenyl)-propionic acid or its metal salt and L-lysine or its acid salt is carried out in a solvent under heat.

7. The process of claim 2 wherein the metal salt, such as the sodium salt, of dl-threo-2-hydroxy-3-(2-aminophenylthio)-3-(4-methoxyphenyl)-propionic acid of formula (II) is reacted with the acid salt, such as the HCl salt, of L-lysine.

8. A process for producing d-threo-2-hydroxy-3-(2-aminophenylthio)-3-(4-methoxyphenyl)-propionic acid represented by the following formula (I)

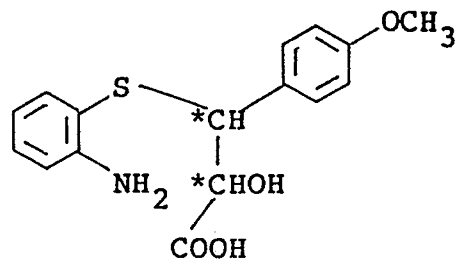

..... (I)

which comprises adding an inorganic acid to an aqueous

(19) Europäisches Patentamt
European Patent Office
Office européen des brevets

(11) Publication number: **0 320 532 A1**

(12) **EUROPEAN PATENT APPLICATION**

(21) Application number: **87118854.6**

(51) Int. Cl.4: **C07C 149/42 , C07B 57/00**

(22) Date of filing: **18.12.87**

A request for correction of some errors in the the originally filed description and claims has been filed pursuant to Rule 88 EPC. A decision on the request will be taken during the proceedings before the Examining Division (Guidelines for Examination in the EPO, A-V, 2.2).

(43) Date of publication of application: **21.06.89 Bulletin 89/25**

(84) Designated Contracting States: **AT BE CH DE ES FR GB GR IT LI LU NL SE**

(71) Applicant: **Nihon Iyakuhin Kogyo Co., Ltd.**
**6-21, Sougawa 1-chome**
**Toyama City Toyama Prefecture(JP)**

Applicant: **SANYO KAGAKU KANKYUSHO CO., LTD.**
**148-1, Tajii Mihara-cho Minamikawachi-gun**
**Osaka-fu(JP)**

(72) Inventor: **Nakamoto, Hiromasa**
**5-26, Marunouchi**
**Takaoka-shi Toyama-ken(JP)**
Inventor: **Ishizuka, Naoyasu**
**5-27, Ohmachi**
**Takaoka-shi Toyama-ken(JP)**
Inventor: **Futsukaichi, Osamu**
**192, Ebiehamabiraki**
**Shinminato-shi Toyama-ken(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Novel optically active carboxylic acid derivative l-lysine salt and process for production thereof.**

(57) A novel salt of d-threo-2-hydroxy-3-(2-amino-phenylthio)-3-(4-methoxyphenyl)-propionic acid represented by the following formula (1)

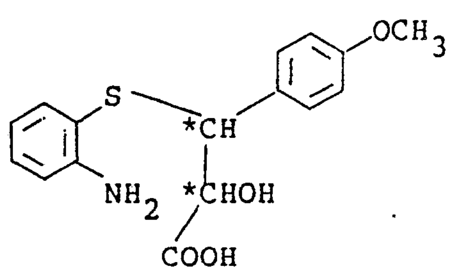

..... (I)

wherein C* represents the carbon atoms of the optically resolved molecules, with L-lysine. The above compound can be produced by reacting d1-threo-2-hydroxy-3-(2-aminophenylthio)-3-(4-methoxyphenyl)-propionic acid with L-lysine or a metal salt of said propionic acid with an acid salt of L-lysine to form corresponding salts, and separating the difficultly-soluble salt from the resulting two optically

EP 0 320 532 A1

active salts by utilizing the difference in solubility in a solvent between the two optically active salts. This compound is useful as a synthesis intermediate for a known pharmaceutical compound, diltiazem hydrochloride.

This invention relates to L-lysine d-threo-2-hydroxy-3-(2-aminophenylthio)-3-(4-methoxyphenyl)-propionate which is useful as a synthesis intermediate for diltiazem hydrochloride of formula (VI) given hereinafter, a known pharmaceutical compound useful for treatment of, for example, angina pectoris and essential hypertension, and which is not described in the prior literature; and to a process for its production. The invention also relates to a process for producing a pharmaceutical compound of formula (VI) by utilizing this novel optically active carboxylic acid derivative salt.

More specifically, this invention relates to a salt between d-threo-2-hydroxy-3-(2-aminophenylthio)-3-(4-methoxyphenyl)-propionic acid represented by the following formula (I)

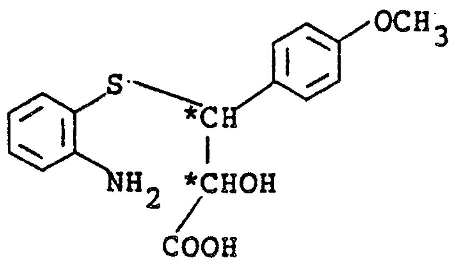

..... (I)

wherein *C represents the carbon atoms of the optically resolved molecules,

and L-lysine.

This invention also relates to a process for producing L-lysine d-threo-2-hydroxy-3-(2-aminophenyl-thio)-3-(4-methoxyphenyl)-propionate, which comprises reacting d1-threo-2-hydroxy-3-(2-aminophenylthio)-3-(4-methoxyphenyl)-propionic acid represented by the following formula (II)

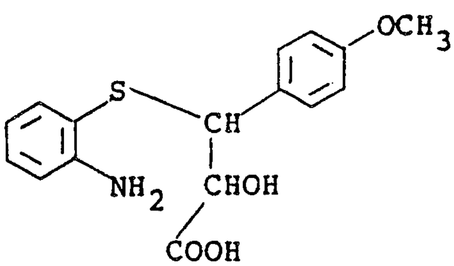

..... (II)

with L-lysine or a metal salt of said propionic acid with an acid salt of L-lysine to form corresponding salts, and separating the difficultly-soluble salt from the resulting two optically active salts by utilizing the difference in solubility in a solvent between the two optically active salts.

A known compound, d-3-acetoxy-cis-2,3-dihydro-5-[2-(dimethylamino)ethyl]-2-(4-methoxyphenyl)-1,5-benzothiazepin-4(5H)-one hydrochloride, represented by the following formula (VI)

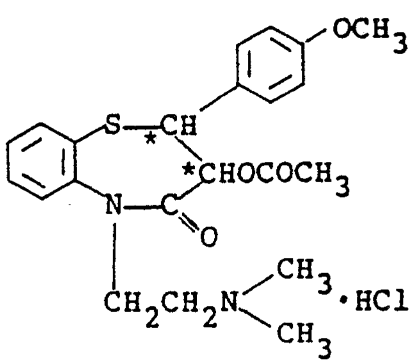

..... (VI)

is a known pharmaceutical compound also called "diltiazem hydrochloride". It is useful for alleviating pains

in angina of effort and obsolete myocardial infarction and treatment of essential hypertension [Manual of Newly Developed Pharmaceuticals (a Japanese-language publication), 3rd edition, page 543, 1983].

Methods have been known to produce d-threo-2-hydroxy-3-(2-aminophenylthio)-3-(4-methoxyphenyl)-propionic acid useful for the production of diltiazem hydrochloride (d-isomer) useful as the aforesaid therapeutic agent by optically resolving the known intermediate compound of formula (II), i.e. d1-threo-2-hydroxy-3-(2-aminophenyl-thio)-3-(4-methoxyphenyl)-propionic acid. For example, Japanese Laid-Open Patent Publication No. 231065/1984 discloses a method in which optically active alpha-phenyl-ethylamine is used as an optical resolving reagent. Japanese Laid-Open Patent Publication No. 69756/1986 discloses a method in which L-1,2-diphenylethylamine is used as an optical resolving reagent. However, the optical resolving reagents used in these methods are expensive and difficult to procure, and are unsuitable for use in industrial practice.

The present inventors have made investigations in order to develop a technique which can overcome this disadvantage. These investigations have unexpectedly led to the discovery that L-lysine or its acid salt such as L-lysine hydrochloride which is very cheap in price and easily available reacts easily with d1-threo-2-hydroxy-3-(2-aminophenylthio)-3-(4-methoxyphenyl)-propionic acid of formula (II) or its metal salt to form two optically active salts and effectively form the wanted difficultly-solvent-soluble salt of d-threo-2-hydroxy-3-(4-methoxy-phenyl)-propionic acid and L-lysine while the salt of the 1-isomer remains dissolved in the solvent, and thus the novel salt of d-threo-2-hydroxy-3-(2-aminophenylthio)-3-(4-methoxyphenyl)-propionic acid of formula (1) and L-lysine can be industrially advantageously separated and recovered from the two salts.

It is an object of this invention therefore to provide L-lysine d-threo-2-hydroxy-3-(2-aminophenylthio)-3-(4-methoxyphenyl)-propionate which is useful, for example, as a synthesis intermediate for a known pharmaceutical compound, diltiazem hydrochloride, and which is not described in the prior literature.

Another object of this invention is to provide a process f-or producing the aforesaid salt.

Other objects of this invention along with its advantages will become apparent from the following description.

The novel salt of d-threo-2-hydroxy-3-(2-amino-phenylthio)-3-(4-methoxyphenyl)-propionic acid of formula (I) with L-lysine can be obtained by reacting d1-threo-2-hydroxy-3-(2-aminophenylthio)-3-(4-methoxyphenyl)-propionic acid and L-lysine or a metal salt of said propionic acid with an acid salt of L-lysine to form corresponding salts, and separating the difficultly-soluble salt from the resulting two optically active salts by utilizing the difference in solubility in a solvent between the two optically active salts.

The reaction of the d1-isomer of formula (II) or its metal salt with L-lysine or its acid salt can be carried out by contacting them with each other in a suitable resolving solvent. The resolving solvent may be an aqueous organic solvent, for example mixtures of water with lower alcohols such as methanol, ethanol and isopropanol, and a mixture of acetone and water. The amount of the resolving solvent may be properly varied, and is, for example, about 2 to about 5 times the volume of the d1-isomer of formula (II). The amount of L-lysine used is nearly equivalent, for example about 1 to about 1.2 equivalents, to the d1-isomer of formula (II).

Desirably, the reaction between the d1-isomer of formula (II) or its metal salt with L-lysine or its acid salt is carried out under heat. The two optically active salts formed by the reaction may be allowed to cool, or positively cooled, to crystallize the difficultly-soluble salt by utilizing the difference in solubility in a solvent between the two diastereomers. The difficultlysoluble salt is separated and obtained as L-lysine d-threo-2-hydroxy-3-(2-aminophenylthio)-2-(4-methoxyphenyl)-propionate.

The heating temperature may be a suitable temperature at which the d1-isomer of formula (II) or its metal salt and L-lysine or its acid salt can dissolve in the resolving solvent. Usually, the reaction under refluxing conditions is preferred. Preferably, the reaction is carried out by using a metal salt of the d1-isomer of formula (II) such as its sodium salt and an acid salt of L-lysine such as L-lysine hydrochloride.

By reacting the d1-isomer of formula (II) with L-lysine or the metal salt of the d1-isomer of formula (II) and the acid salt of L-lysine in the manner described above, the corresponding salts are formed. The difficultly-soluble salt, L-lysine d-threo-2-hydroxy-3-(2-aminophenyl-thio)-3-(4-methoxyphenyl)-propionate, is separated by utilizing the difference in solubility in a solvent between the two resulting optically active salts. As desired, the resulting L-lysine salt may be recrystallized from a hydrous lower alcohol, for example, to increase its optical purity further. The L-lysine d-threo-2-hydroxy-2-(2-aminophenylthio)-3-(4-methoxyphenyl)-propionate may be converted to a free acid form of formula (I) by dissolving it in water, adding 10% hydrochloric acid for example, to adjust the pH of the system to about 2.5 to about 3.5, and collecting the precipitated crystals by filtration.

The starting d1-isomer of formula (II) is a known compound and can be produced, for example, by using the process described in Japanese Patent Publication No. 9383/1970.

One embodiment of producing the known pharmaceutical compound of formula (VI) from the L-lysine salt of the compound of formula (I) provided by this invention is schematically shown below.

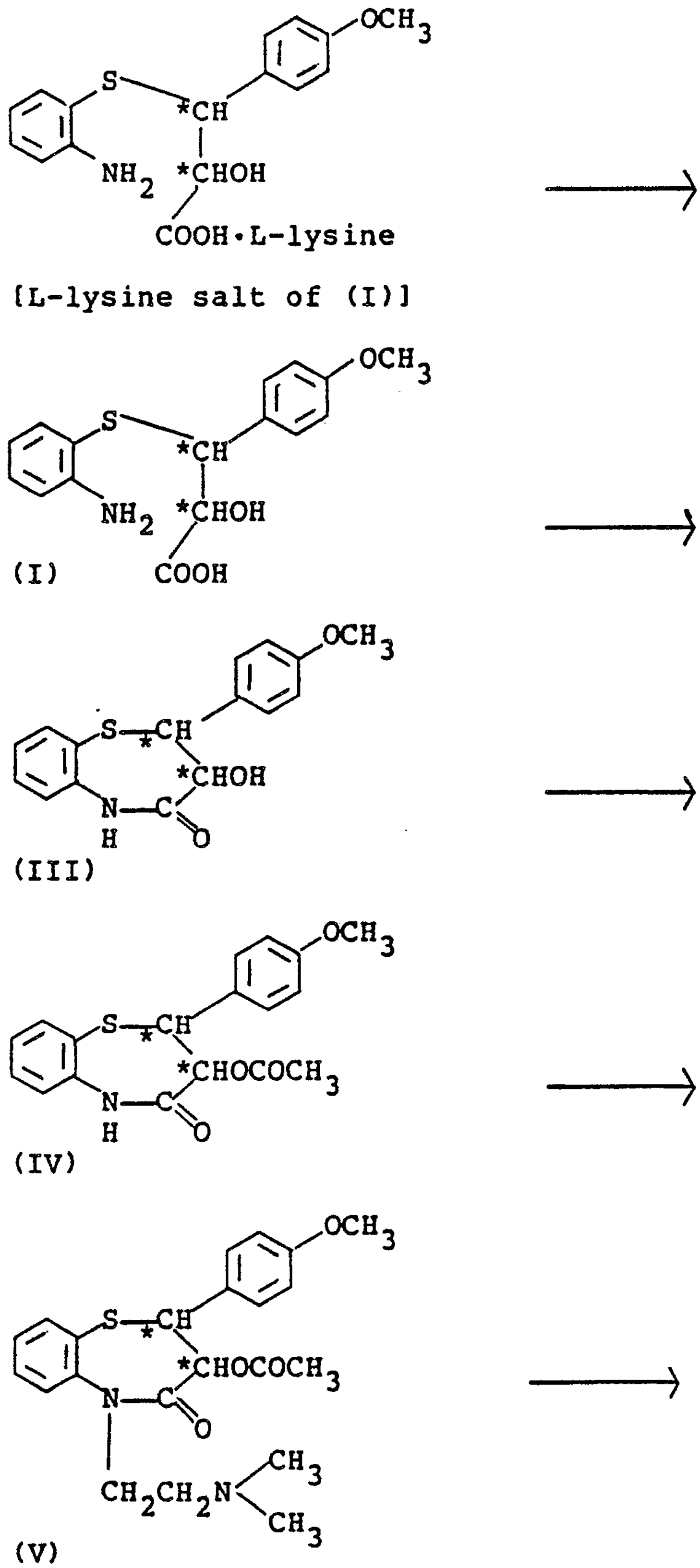

[L-lysine salt of (I)]

(I)

(III)

(IV)

(V)

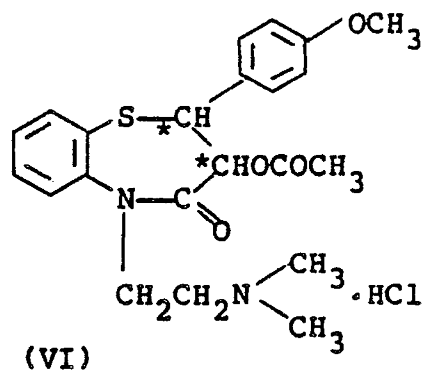

(VI)

In the above scheme, the compound of formula (III) may be obtained from the compound of formula (I) by, for example, using the process described in Japanese Patent Publication No. 8982/1971. The compound of formula (IV) may be obtained from the compound of formula (III) by, for example, using the process described in Japanese Patent Publication No. 43785/1971. The production of the pharmaceutical compound of formula (VI) from the compound of formula (IV) via the compound of formula (V) may be effected by, for example, using the process described in Japanese Laid-Open Patent Publication No. 32777/1985.

EXAMPLE 1

L-lysine d-threo-2-hydroxy-3-(2-aminophenylthio)-3-(4-methoxyphenyl)-propionate:-

To a solution of 27.5 g of sodium hydroxide in 140 ml of water were added 211 g of d1-threo-2-hydroxy-3-(2-aminophenylthio)-3-(4-methoxyphenyl)-propionate and 400 ml of methanol, and the mixture was heated to form a solution. Then, 122 g of L-lysine hydrochloride was added to the solution, and the mixture was heated under reflux. The solution was allowed to cool to room temperature. The precipitated crystals were collected by filtration, washed with methanol, recrystallized from 50% hydrous methanol, and dried to give 146.5 g of L-lysine d-threo-2-hydroxy-3-(2-aminophenylthio)-3-(4-methoxyphenyl)-propionate.
Yield: 95.2%
$[\alpha]_D^{20}$ : +252.3° (c=0.5, water)
Melting point: 138-140°C

EXAMPLE 2

d-Threo-2-hydroxy-3-(2-aminophenylthio)-3-(4-methoxyphenyl)-propionic acid:-

146 g of the salt product obtained in Example 1 was dissolved in 500 ml of water and the solution was adjusted to pH 3.0 with 10% hydrochloric acid. The precipitated crystals were collected by filtration, washed with water, and dried to give 71.7 g of optically pure d-threo-2-hydroxy-3-(2-aminophenylthio)-3-(4-methoxyphenyl)-propionic acid.
Yield: 68.1%
$[\alpha]_D^{20}$ : +350°C (c=0.356, methanol)
Melting point: 137.5°C

EXAMPLE 3

L-lysine d-threo-2-hydroxy-3-(2-aminophenylthio)-3-(4-methoxyphenyl)-propionate:-

32 g of d1-threo-2-hydroxy-3-(2-aminophenylthio)-3-(4-methoxyphenyl)-propionic acid and 15.2 g of L-lysine were added to 35 ml of water and 100 ml of methanol. The mixture was heated to form a solution. The solution was allowed to cool to room temperature. The precipitated crystals were collected by filtration, washed with methanol, recrystallized from 50% hydrous methanol, and dried to give 20.5 g of L-lysine d-threo-2-hydroxy-3-(2-aminophenylthio)-3-(4-methoxyphenyl)-propionate.
Yield: 87.8%
$[\alpha]_D^{20}$ : +252.0 (c=0.5, water)
Melting point: 138-140 °C

## Claims

1. A salt of d-threo-2-hydroxy-3-(2-aminophenylthio)-3-(4-methoxyphenyl)-propionic acid represented by the following formula (I)

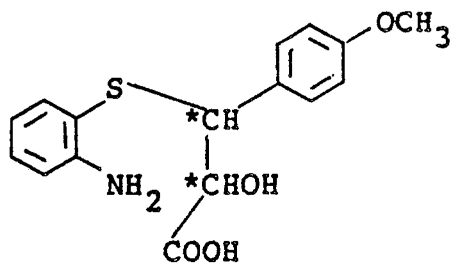

..... (I)

wherein C* represents the carbon atoms of the optically resolved molecules,

with L-lysine.

2. A process for producing a salt of d-threo-2-hydroxy-3-(2-aminophenylthio)-3-(4-methoxyphenyl)-propionic acid represented by the following formula (I)

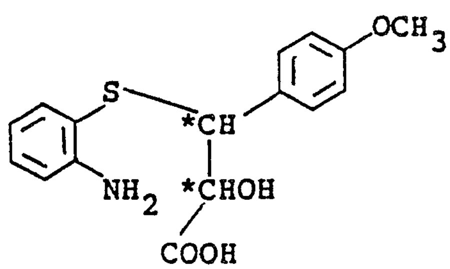

..... (I)

wherein C* represents the carbon atoms of the optically resolved molecules,

with lysine, which comprises reacting d1-threo-2-hydroxy-3-(2-aminophenylthio)-3-(4-methoxyphenyl)-propionic acid represented by the following formula (II)

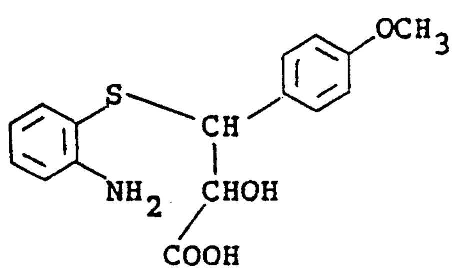

..... (II)

with L-lysine or a metal salt of said propionic acid with an acid salt of L-lysine to form corresponding salts, and separating the difficultly-soluble salt from the resulting two optically active salts by utilizing the difference in solubility in a solvent between the two optically active salts.

3. The process of claim 2 wherein the solvent is an aqueous organic solvent.

4. The process of claim 3 wherein the aqueous organic solvent is a mixture of a lower alcohol and water, or a mixture of acetone and water.

5. The process of claim 2 wherein about 1 to about 1.2 equivalents of lysine or its acid salt is used per equivalent of d1-threo-2-hydroxy-3-(2-aminophenylthio)-3-(4-methoxyphenyl)-propionic acid of formula (II) or its metal salt.

6. The process of claim 2 wherein the reaction of dl-threo-2-hydroxy-3-(2-aminophenylthio)-3-(4-methoxyphenyl)-propionic acid or its metal salt and L-lysine or its acid salt is carried out in a solvent under heat.

7. The process of claim 2 wherein the metal salt, such as the sodium salt, of d1-threo-2-hydroxy-3-(2-aminophenylthio)-3-(4-methoxyphenyl)-propionic acid of formula (II) is reacted with the acid salt, such as the HCl salt, of L-lysine.

8. A process for producing d-threo-2-hydroxy-3-(2-aminophenylthio)-3-(4-methoxyphenyl)-propionic acid represented by the following formula (1)

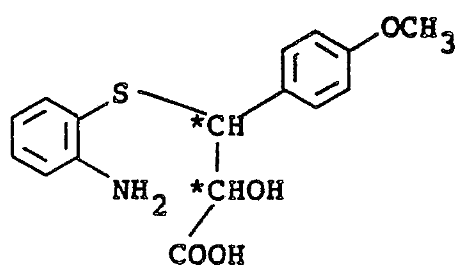

..... (I)

which comprises adding an inorganic acid to an aqueous solution of a salt of the d-threo-2-hydroxy-3-(2-amino-phenylthio)-3-(4-methoxyphenyl)-propionic acid of formula (1) with L-lysine to adjust the pH of the solution to about 2.5 to about 3.5 thereby to precipitate d-threo-2-hydroxy-3-(2-aminophenylthio)-3-(4-methoxyphenyl)-propionic acid from the aqueous solution.

European Patent Office

# EUROPEAN SEARCH REPORT

Application Number

EP 87 11 8854

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 534 579 (ISTITUTO LUSO FARMACO D'ITALIA S.p.A.)<br>* Whole document *<br>----- | 1-8 | C 07 C 149/42<br>C 07 B 57/00 |
| | | | **TECHNICAL FIELDS SEARCHED (Int. Cl.4)** |
| | | | C 07 C 149/00 |

The present search report has been drawn up for all claims

| Place of search | Date of completion of the search | Examiner |
|---|---|---|
| THE HAGUE | 18-08-1988 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)